# EUROPEAN PATENT SPECIFICATION

(11) **EP 3 931 344 B1**
(45) Date of publication and mention of the grant of the patent: **04.09.2024**
(21) Application number: 20703161.8
(22) Date of filing: 24.01.2020
(51) Int. Cl.: C12Q 1/02, G16H 10/40, G16H 30/40, G16H 40/67, G16H 50/20, G16H 50/70

(54) **METHOD AND SYSTEM FOR DETECTION OF MICRO ORGANISMS**
VERFAHREN UND SYSTEM ZUR DETEKTION VON MIKROORGANISMEN
PROCÉDÉ ET SYSTÈME DE DÉTECTION DE MICRO-ORGANISMES

(30) Priority: 01.03.2019 SE 1950267
(43) Date of publication of application: 05.01.2022
(73) Proprietor: Agricam Aktiebolag, 583 30 Linköping (SE)
(72) Inventor: EINEREN, Ellinor, 582 34 Linköping (SE); JOHANSSON, Martin, 582 36 Linköping (SE); TUOMA, Yousif, 582 21 Linköping (SE); GHEEL, David, 582 16 Linköping (SE); FORSGREN, Olliver, 541 31 Skövde (SE)
(74) Representative: AWA Sweden AB
(86) International application number: PCT/EP2020/051718
(87) International publication number: WO 2020/177943

(56) References cited:
- GUOAN ZHENG ET AL: "The ePetri dish, an on-chip cell imaging platform based on subpixel perspective sweeping microscopy (SPSM)", PROCEEDINGS OF THE NATIONAL ACADEMY OF SCIENCES OF THE UNITED STATES OF AMERICA, 11 October 2011 (2011-10-11), United States, pages 16889 - 16894, XP055185863, Retrieved from the Internet <URL:http://www.jstor.org/stable/41321803> DOI: 10.1073/pnas.1110681108
- CALTECH: "Caltech Engineers Build Smart Petri Dish", 3 October 2011 (2011-10-03), pages 1, XP054980379, Retrieved from the Internet <URL:https://www.youtube.com/watch?v=v7h3rf1lrdg> [retrieved on 20200409]
- JAIR C. FERREIRA ET AL: "Comparative analysis of four commercial on-farm culture methods to identify bacteria associated with clinical mastitis in dairy cattle", PLOS ONE, vol. 13, no. 3, 15 March 2018 (2018-03-15), pages e0194211, XP055684697, DOI: 10.1371/journal.pone.0194211
- M. OGAWA ET AL: "Multicolour digital image analysis system for identification of bacteria and concurrent assessment of their respiratory activity", JOURNAL OF APPLIED MICROBIOLOGY., vol. 98, no. 5, 1 May 2005 (2005-05-01), GB, pages 1101 - 1106, XP055684667, ISSN: 1364-5072, DOI: 10.1111/j.1365-2672.2005.02551.x
- EVGENY PUCHKOV: "Image Analysis in Microbiology: A Review", JOURNAL OF COMPUTER AND COMMUNICATIONS, vol. 04, no. 15, 1 January 2016 (2016-01-01), pages 8 - 32, XP055597013, ISSN: 2327-5219, DOI: 10.4236/jcc.2016.415002
- ANDREINI PAOLO ET AL: "Automatic image classification for the urinoculture screening", COMPUTERS IN BIOLOGY AND MEDICINE, NEW YORK, NY, US, vol. 70, 7 January 2016 (2016-01-07), pages 12 - 22, XP029418974, ISSN: 0010-4825, DOI: 10.1016/J.COMPBIOMED.2015.12.025
- WEI-BANG CHEN ET AL: "An automated bacterial colony counting and classification system", INFORMATION SYSTEMS FRONTIERS ; A JOURNAL OF RESEARCH AND INNOVATION, KLUWER ACADEMIC PUBLISHERS, BO, vol. 11, no. 4, 18 February 2009 (2009-02-18), pages 349 - 368, XP019730180, ISSN: 1572-9419, DOI: 10.1007/S10796-009-9149-0
- HIRAKU SASAKI ET AL: "Analysis of the Structure of the Bacterial Community in the Livestock Manure-based Composting Process", ASIAN-AUST. J. ANIM. SCI., 1 January 2009 (2009-01-01), pages 113 - 118, XP055684884, Retrieved from the Internet <URL:https://www.ajas.info/upload/pdf/22-15.pdf> [retrieved on 20200409]

## Description

### Technical field

The present document relates to a method, a device and a system for detection of microorganisms in milk.

### Background

One challenge for dairy farmers is to handle the occurrence of microbial infections in the animals, and in particular in the milk they produce. A conventional way of determining presence of microbes in milk is to collect a milk sample from an animal and to send this sample for testing.

Another challenge for livestock farmers in general, is the occurrence of certain pathogens in livestock, their feed or their environment. Pathogens, such as salmonella and EHEC, which may be found in the animals' feed, inner organs and in the manure, may be particular causes for concern. A conventional way of determining presence of the pathogens is to collect a sample of manure from an animal or a herd and send this sample for testing. Another way is to collect a sample from an autopsy of an animal and send this sample for testing.

For the purpose of this document, the term "livestock" includes, but is not limited to, cattle, pigs, sheep and poultry, regardless of whether such livestock is kept for production of milk, meat, hide or other purposes.

The test procedure generally involves applying the sample on an agar plate, storing the agar plate for a time sufficient to allow bacterial growth to form and then to have a trained expert determine what microbes were present in the sample.

Based on such determination, actions to be taken can be determined, such as to administer antibiotics.

Unfortunately, the procedures outlined above are slow, not only due to the time it takes for the bacterial growth to form, but also due to the time it takes to ship the sample, in the sample waiting to be assessed, e.g. due to backlogs, and in the work to be performed for entering sample results for reporting.

The references Guoan Zheng ET AL, PNAS, 2011, pp. 16889-16894 and "Caltech Engineers Build Smart Petri Dish", https://www.youtube.com/watch?v=v7h3rf1lrdg disclose the use of a separate image sensor for capturing images of a growth medium test plate (ePetri dish).

JAIR C. FERREIRA ET AL, PLOS ONE, vol. 13, no. 3, 2018, page e0194211, discloses a method of processing a milk sample obtained from a livestock animal, comprising a microscopic and Gram stain evaluation and classifying the micro-organism based thereon.

M. OGAWA ET AL, JOURNAL OF APPLIED MICROBIOLOGY., vol. 98, no. 5, 2005, pages 1101-1106 discloses a multicolour digital image analysis system and method for the identification of bacteria in a milk sample obtained from a livestock animal based on a visual spectrum image depicting at least part of the test surface.

EVGENY PUCHKOV, JOURNAL OF COMPUTER AND COMMUNICATIONS, vol. 04, no. 15, 2016, pages 8-32; ANDREINI PAOLO ET AL, COMPUTERS IN BIOLOGY AND MEDICINE, vol. 70, 2016, pages 12-22 and WEI-BANG CHEN ET AL, INFORMATION SYSTEMS FRONTIERS ; A JOURNAL OF RESEARCH AND INNOVATION, vol. 11, no. 4, 2009, pages 349-368 all disclose a method of training an image classifier algorithm for determining a micro-organism type based on a micro-organism growth pattern depicted in a visible spectrum image.

Hiraku Sasaki ETAL, Asian-Aust. J. Anim. Sci., 2009, pages 113-118 discloses PCR based analysis of the Structure of a bacterial community in livestock manure.

There is a need for an improved method of detecting microorganisms in farm animals.

### Summary

The waiting time may be determined as a predetermined time period, such as 12-48 hours, 12-36 hours, and preferably about 24 hours. Alternatively, the waiting time may be determined based on a growth amount.

The term "visual spectrum" implies that the image contains a spectrum that is visible to the human eye, which normally comprises wavelengths of about 380 to 740 nanometers.

A "growth pattern" is combination of shape(s) and colors that is provided by the microorganism(s) as they grow and form colonies on the growth medium.

Applicant's tests reveal that it is possible to train an image classifier algorithm to a level where the ability to correctly determine a microbe type based on a visual spectrum image of the test plate is comparable to that of a trained expert.

Hence, the method provides a user friendly way of determining presence and type of microbes in milk samples. Moreover, the method can be implemented at a substantially reduced cost compared and the availability of testing capacity can be greatly increased, with a reduction in test lead times being reduced.

Moreover, the method is implemented with hardware that is readily available to most people, such as a smartphone or a tablet, or which can be provided at low cost.

The test plate may comprise at least two juxtaposed growth medium regions, said regions differing in at least one of type, color, concentration and composition of the respective growth medium.

In particular, the test plate may present 1-10 such different growth medium regions, preferably 2-4 different growth medium regions.

The method may further comprise arranging the test plate with a predetermined orientation relative to the image capture device prior to acquiring said image, such that the growth medium regions present a predetermined orientation in said image; and/or reorienting the acquired image, such that the growth medium regions present a predetermined orientation in said image.

The waiting step may comprise maintaining the sample in a temperature controlled environment, preferably at a constant temperature of 34-40 degrees C.

The image capture device forms part of a smartphone or a tablet.

The method comprises positioning the test plate on a first part of an image capture support and positioning the image capture device on a second part of the image capture support, said second part being spaced from the first part, wherein the acquisition of the image is performed while the image capture device and the test plate are positioned on the image capture support.

The method may further comprise enclosing the test plate so as to shield it from ambient light and supplying light from a light source, optionally via a reflector. The light source may be provided inside an enclosure.

The image capture support may be an image capture support as described in the present document.

The method may further comprise an image limitation step, comprising cropping or masking unwanted portions of the image.

For example, the image which is sent may be the originally captured image or a processed version of the image, such as a partially cropped or masked image.

The pre-trained image classifier algorithm may comprise at least one supervised learning algorithm configured and trained to identify at least two microorganism types or microorganism classes.

Examples of supervised learning algorithms include, but are not limited to, convolutional neural networks, decision trees (such as random forest), support vector machine and fully connected neural network.

Alternatively, or as a supplement, the pre-trained image classifier algorithm may comprise a plurality of supervised learning algorithms, each of which being configured and trained to identify one microorganism type or microorganism class.

Said applying an image classifier algorithm may comprise sending the image from the image capture device via a data communication network to a remotely located processing device, feeding said image to the pre-trained image classifier to obtain a processing result based on the image, and sending the processing result via the data communication network to the image capture device or to another processing device.

The other processing device may be a further user device, such as a smartphone or tablet; a user computer, a web page or a web portal, a veterinarian's computer, etc.

The processing result may comprise an indication of a microorganism type deemed to be present on the test plate depicted on the image, and optionally a value indicating a confidence level of the processing result.

The method may further comprise waiting for a second time sufficient to allow further microbial growth to form on said test surface, acquiring a second visual spectrum image of the test surface using an image capture device, and applying the image classifier algorithm to said second image in order to determine a microorganism type based on a microorganism growth pattern visible on the image.

The second waiting time may be determined as a predetermined time period, such as 12-48 hours, 12-36 hours, and preferably about 24 hours. Alternatively, the waiting time may be determined based on a growth amount.

It is possible to apply further cycles of waiting and acquiring further images that are processed by being applied to the image classifier algorithm, in the manner described above.

The sample may be a milk sample from a lactating animal. In this case, the milk may be applied directly to the test surface.

Alternatively, the sample may be a manure sample from an animal. In this case, the sample may be applied directly to the test plate. Alternatively, the manure sample may be diluted, dissolved or suspended in a liquid, such as water, whereby such dilution, solution or suspension is applied to the test surface.

It is also possible to sample the animals' feed, in the same manner as manure, and in particular by first diluting, dissolving or suspending the feed in a liquid and then applying that liquid to the test surface.

According to a second aspect, there is provided a system as defined in claim 14 for processing a sample obtained from a livestock animal, comprising a growth medium test plate, an image capture support as described above, a user device comprising an image capture device as defined in claim 14 and a communication device, and a central processing device, wherein the user device is configured acquire a visual spectrum image depicting at least part of a test surface of the growth medium test plate, using the image capture device, and to send the acquired image to the central processing device, and wherein the central processing device is configured to receive the image, provide a computer-implemented pre-trained image classifier algorithm, said image classifier algorithm being pre-trained to determine a microorganism type based on a visible spectrum image depicting a growth pattern of a known microorganism, and apply the image to the pre-trained image classifier algorithm to determine a microorganism type based on a microorganism growth pattern visible on the image.

### Brief description of the drawings

Fig. 1 is a schematic diagram of a system in which the present concept can be implemented.
Fig. 2 is a schematic diagram of a user device.
Fig. 3 is a schematic flowchart of a method according to the present concept.
Figs 4a-4b schematically illustrate an image capture support.
Figs 5a-5b schematically illustrate the image capture support with a user device positioned therein.

### Detailed description

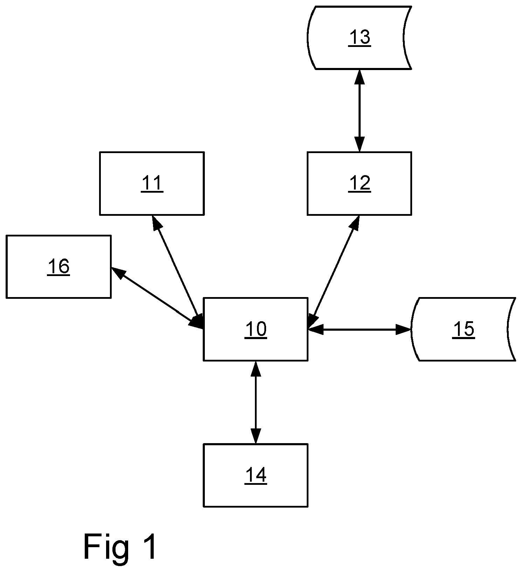

Fig. 1 schematically illustrates a non-limiting diagram of a system in which the present concept can be implemented.

The system may comprise a central processing unit 10, a user device 11; a veterinarian work station 12, which is connected to a journal storage 13; an image classifier subsystem 14 and a data storage unit 15. The system may comprise further user devices and one or more user work stations 16.

The central processing 10 unit may be implemented as a server, such as a web server, with storage and processing capability. The central processing unit may comprise the image classifier subsystem 14 and the storage unit 15.

The storage unit 15 may store image data and data relating to such images. The identifiers may include one or more of position coordinates, farm id, user id, animal id, teat id, date and time.

The central processing unit 10 may thus run software for receiving data for communicating with the user device(s) 11, 16, the veterinarian work station 12, and for implementing the image classifier subsystem 14 and the storage unit 15.

Alternatively, the central processing unit may be implemented as a cloud device.

Further, the image classifier subsystem 14 and/or the storage unit 15 may be implemented as cloud devices.

The veterinarian work station 12 may comprise a journal storage 13 for storing general veterinarian records relating individual animals. Such records may be supplemented by image data, corresponding to what is stored at the storage unit 15. Alternatively, or additionally, the records may merely be supplemented by processing results from the central processing unit 10, as will be described in the following.

The image classifier subsystem 14 can be provided in the form of a supervised learning algorithm that may be implemented in the form of a neural network, such as CNN (Convolutional Neural Network) or RNN (Recurring Neural Network).

The image classifier subsystem 14 needs to be trained, which can be achieved by inputting a number of images of test plates with bacterial growth, which images each is associated with one or more microbe types, as identified by expert users and/or by DNA analysis.

Such image classifier subsystems 14 are known and available as open source software. Alternatively, the image classifier may be implemented in the central processing unit 10.

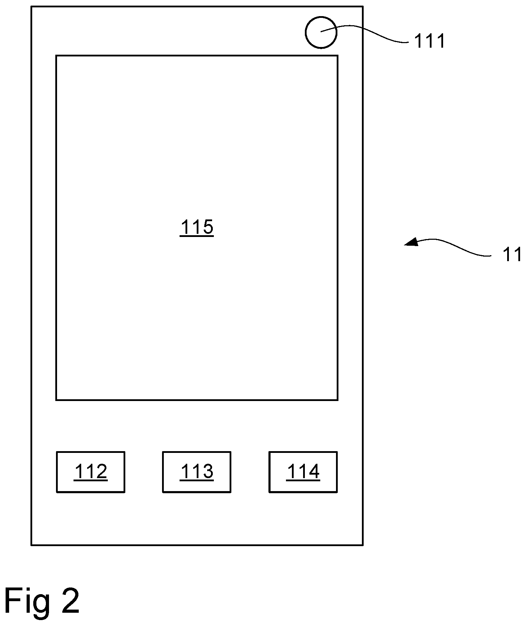

Referring to fig. 2, the user device(s) 11 may take the form of a smart phone or tablet, which comprises an image capture device 111, a processing device 112, a memory 113, a communication device 114 and a user interface 115. The user device 11 may run software for implementing related parts of the method disclosed herein and for communicating with the central processing unit 10.

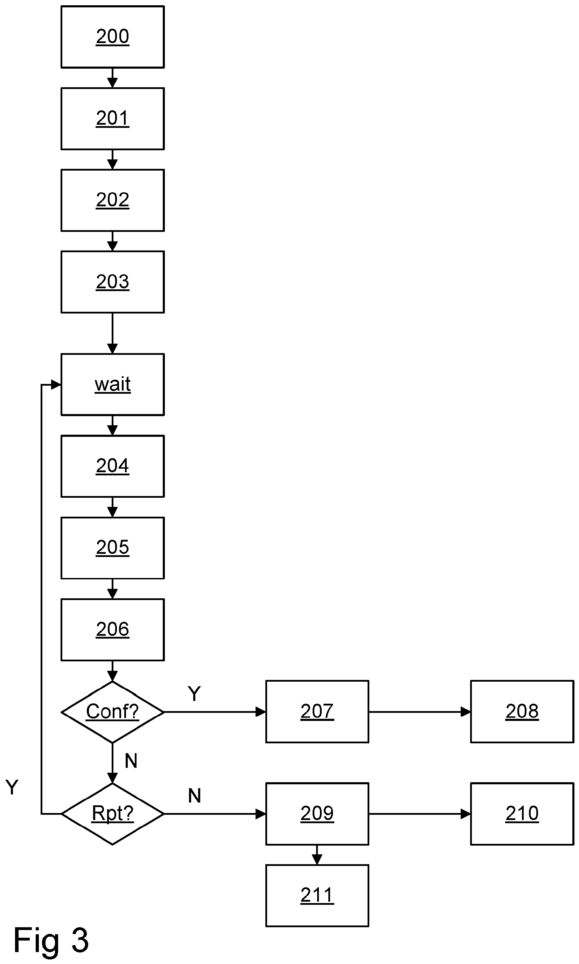

Fig. 3 schematically illustrates a flowchart of a method in which the present concept can be implemented.

The method is described with reference to a milk sample. However, a manure sample may be processed in the same way, with the possible modification that a manure sample may, depending on its texture or viscosity, be diluted, dissolved, or suspended in a liquid, such as water, prior to its application to the test surface.

In step 200, a new sample operation is initiated. This step may be preceded by a detection of an anomaly relating to the animal, e.g. in accordance with the disclosure of WO2012080275A1. Such anomaly detection may then trigger the acquisition of a milk sample.

In step 201, an animal id is entered, e.g. scanned from a tag on the animal or manually input.

In step 202, a teat id is entered, e.g. manually entered by selection from a schematic image on the user interface.

In step 203, a test tube id is entered, e.g. scanned or manually input.

In connection with step 203, the udder is prepared, such as cleaned and a sample is collected in the test tube. These steps are typically performed in the direct vicinity of the animal.

The steps above are typically performed using a user device 11 in the form of a smartphone or a tablet.

The following steps may be performed in a designated area, such as a local lab or in a local control central.

In particular, milk from the test tube is transferred onto a test plate and the test tube id is also transferred to the test plate, e.g. by peeling off an id carrying sticker from the test tube and attaching the sticker to the test plate, or by associating a pre-provided test plate id with the test tube id.

After step 203, the test plate may be stored for a predetermined time, such as 12-48 hours, preferably 24 hours, and preferably in a controlled environment, e.g. in a controlled temperature.

In step 204, the sample is removed from storage, any lid provided on the test plate may be removed and the test plate is positioned on an image capture support, after which a first image is captured by means of the user device 11 (the same user device as before, or another user device having the same functionality) and sent to the central processing device 10.

Before step 204, the user device that is to be used for image capture may need to be initialized, e.g. by entering or scanning test plate id. Alternatively, if the test plate id is visible when the test plate is positioned in the image capture support, the identification and thus initialization may be performed in the same step as the image capture.

In step 205, the image, or a limited version of the image, such as a cropped or masked version of the image, is sent to the central processing device 10. The image may be sent together with data identifying the farm, the animal and the individual teat and a time and date stamp.

Analysis is then carried out in the central processing device 10, wherein the pre-trained image classifier algorithm determines the type(s) of microorganisms preset on the test plate based on the visible spectrum image. The image classifier algorithm may also determine a confidence level, i.e. a value indicating to what extent the analysis can be expected to be reliable.

In step 206, results are received from the central processing device 10. The results may comprise an indication of one or more microbe types found to be present on the test plate when the image was captured, along with a measure of the confidence level of said result.

If the result has a sufficient level of confidence, the result may be presented in step 207 to the user, for example via the user device 11. Such presentation may include an indication of microbe type and optionally an indication of action to be taken, such as what antibiotic to administer.

Optionally, statistical data based on the test and other tests may be presented to the user in step 208.

If the result does not have a sufficient level of confidence (step Conf?), and it is determined in that another growth cycle should be performed (step Rpt?), the user may be prompted to return the test plate to the storage and wait for another predetermined amount of time, such as 12-48 hours, preferably 24 hours, and preferably in a controlled environment, e.g. in a controlled temperature.

After waiting, a second image may be captured in step 204 by means of the user device 11 and sent in step 205 to the central processing device 10.

In step 206, results are again received from the central processing device 10. The results may comprise an indication of one or more microbe types found to be present on the test plate when the image was captured, along with a measure of the confidence level of said result.

If the result is determined (step Conf?) to have a level of confidence, which is not sufficiently high, and it is determined (step Rpt?) that no more growth cycles should be performed, the first and/or the second image may be sent to an evaluator, such as a veterinarian or other expert for a manual assessment in step 209. Such manual classification may be based on visual inspection of the test plate by an expert user and/or by chemical or DNA analysis of microbes present on the plate.

If the result now has a sufficient level of confidence, the result may be presented in step 210 to the user, for example via the user device 11. Such presentation may include an indication of microbe type and optionally an indication of action to be taken, such as what antibiotic to administer.

The outcome of the manual assessment made in step 209 may be forwarded in step 211 to the image classifier 15 for further training of the image classifier.

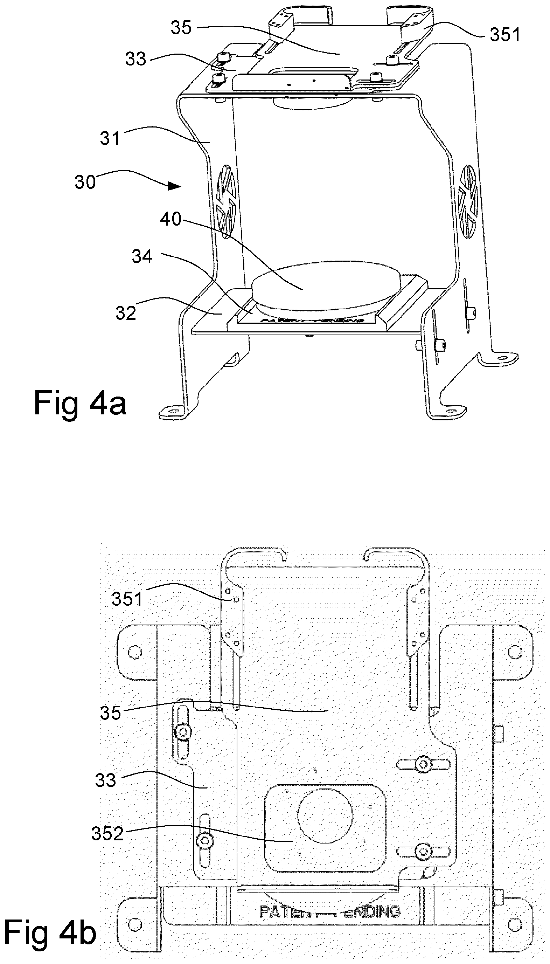

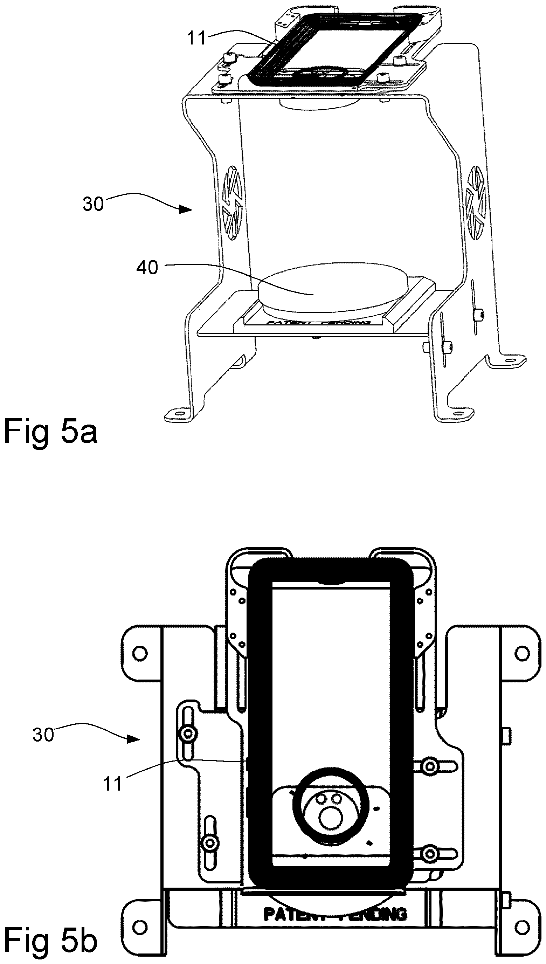

Referring to figs 4a-4b and 5a-5b, the image capture support 30 will now be described.

The image capture support comprises a pair of vertical members 31, a first horizontal member 32 and a second horizontal member 33.

The first horizontal member 32 is used as a test plate support and the second horizontal member 33 is used as an image capture device support. In the illustrated example, the first horizontal member 32 is positioned at a lower vertical level than the second horizontal member 33.

In the illustrated example, the first horizontal member 32 is provided with a test plate holder 34, which is a holder device that is adapted specifically to receive a test plate 40. Preferably, the test plate holder 34 presents a vertical support surface and edges that ensure correct positioning of the test plate 40. Preferably, the edges should ensure correct position in at least two mutually orthogonal directions. In the illustrated example, three edges are provided, thus ensuring correct positioning of the test plate 40 in three directions.

The edges may be designed such that a standardized test plate 40 fits snugly within the edges, with no, or very little play.

Alternatively, the edges may be designed such that the standardized test plate 40 is press fit between at least one pair of opposing edges. To this end, the test plate holder 34 may be at least partially formed of an elastic material.

As illustrated, the vertical position of the first horizontal member may be adjustably attached to the vertical member 31.

The second horizontal member 33 is positioned above the first horizontal member 32. In the illustrated example, the second horizontal member 33 is provided with a holder device 35 that is adapted to receive a user device in the form of a smartphone. To this end, the holder device 35 may present edges 351 designed to ensure that the user device is positioned in the correct position every time it is placed in the holder 35.

The holder device, and thus also the second horizontal member 33 may further comprise a window 352, which is positioned and adapted such that the user device can be positioned with its user interface facing upwardly and its camera facing downwardly, towards the first horizontal member 32.

The holder device 35 may be designed such that its edges are horizontally movable to enable the holder device to snugly accommodate user devices of different sizes and with different camera positions.

Hence, in the illustrated example, the test plate 40 is to be positioned on the first horizontal member 32 with its test surface facing upwardly and the user device is to be positioned on the second horizontal member 33 with its camera facing downwardly towards the test plate 40.

One or more light sources (not shown) can be provided on the image capture support 30.

As a first example, a downwardly illuminating light source may be provided on the underside of the second horizontal member 33 and directed towards the test plate holder 34.

As a second example, an upwardly illuminating light source may be provided on the underside of the test plate holder 34, so as to provide back lighting of the test plate when it is positioned in the test plate holder 34.

One or both light sources may be a white light source having a fixed or tunable color temperature. Alternatively, the light source may be an adjustable light source, that is capable of providing a range of colors by color mixing, such as an RGB type light source. A combination of an RGB and a tunable white light source may be provided.

The light source(s) may be configured for being controlled by the user device 11. For example, the light sources may be activated in a predetermined sequence for providing front lit and back lit versions of an image. As another example, a light source may be activated in a specific sequence in order to provide an image sequence with different light colors or color temperatures.

Communication between the user device and the light source(s) may be through short range radio frequency, such as wifi or Bluetooth, or through cable.

Power supply for the light sources may be from the user device or from a separate power supply.

The image capture support 30 may comprise one or both of such light sources.

The light sources may be designed to provide light in the visible spectrum, and in particular white light. Optionally, the light may be tunable white light.

Referring to figs 5a-5b, there is illustrated the image capture support 30 with a user device in the form of a smartphone 11 received in the image capture device holder 35 and a test plate 40 received in the test plate holder 34.

The image capture support 30 may be adapted to space the image capture device 11 and the test plate 40 on the order of 5-30 cm from each other, preferably 10-20 cm.

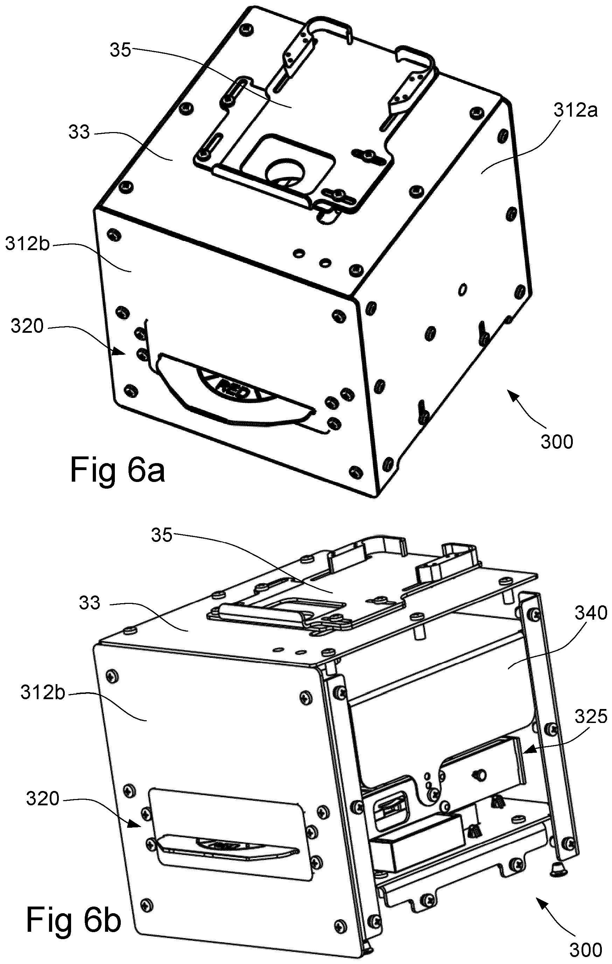

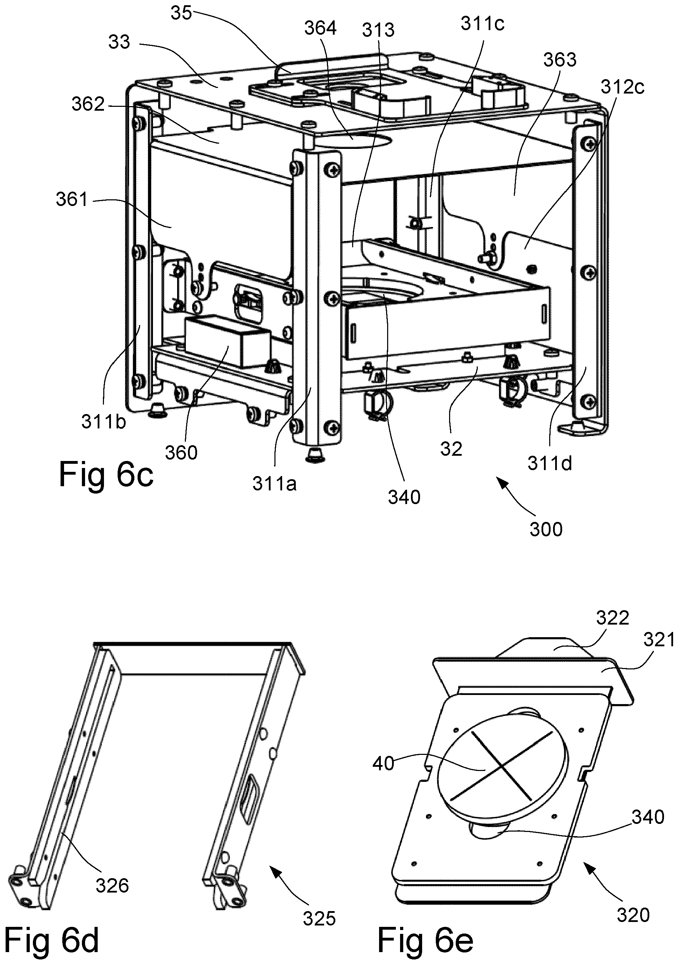

Figs 6a-6e schematically illustrate another embodiment of an image capture support 300, which differs from the image capture support 30 in that the sample holder is enclosed, so as to reduce the impact of ambient light conditions on the image capture process.

Just like the image capture support 30, the image capture support 300 presets vertical members 311a, 311b, 311c, 311d, a first horizontal member 32 and a second horizontal member 33. The second horizontal member 33 supports an image capture device holder 35, which may be designed as described above.

The image capture support 300 presents vertical walls 312a, 312b, 312c, surrounding the sample holder so as to shield it from laterally incoming light. The vertical walls may include a pair of side walls 312a, 312c, a front wall 312b and a rear wall (not shown). One of the walls may comprise an opening 313 through which a sample holder is insertable. In the illustrated example, the opening 313 is provided in the front wall 312b.

The vertical walls may be formed as separate walls that are assembled and attached to the vertical members 311a, 311b, 311c, 311d, as illustrated. Alternatively, the vertical walls may be formed in one piece. As yet another alternative, the vertical walls may provide a self-supporting body, to which the horizontal members 32, 33 are attached.

The sample holder 340 may be provided on a slidable member 320, which is received in a sliding mechanism 325. The slidable member 320 may comprise a front cover plate 321, a handle 322 and a sample holder support 340. The sliding mechanism may comprise horizontal grooves 326, in which edges of the slidable member 320 are slidably received.

A light source 360 may be provided inside a space enclosed by the walls 312a-312c. A reflector 361, 362, 363 may be provided for reflecting the light from the light source towards the sample holder 340. The reflector may comprise two or more portions 361, 362, 363, which extend at an angle relative to each other. The portions 361, 362, 363 may be separate parts or integrated with each other, such as formed in one piece. In the illustrated example, the reflector is formed as a plate comprising a planar central portion 362 having an opening 364 for the optical path of the image capture device 11 and two planar side portions 361, 363, extending at an angle relative to the central portion 362.

The reflector has a reflective surface. The reflective surface may have a mirror finish or a matte finish of a reflective color, such as white or silver, such that the reflected light is diffused for a more even distribution.

The sample holder 300 has the same basic function as the sample holder 30 disclosed above.

In addition, the insertion of the test plate 40 is achieved as follows.

The empty slidable member 320 is slid out of the opening 313, after which a test plate 40 is positioned in the sample holder 340, and the slidable member 320 is slid back through the opening 313. The slidable member 320 may be designed such that, when it is fully inserted, the sample holder 340 is in a predetermined position relative to the image capture device.

The light source 360 is activated, such that light is reflected off the reflector 361, 362, 363 to provide illumination of the test plate 40.

The image capture process is then carried out as described above.

The enclosure, together with the lighting arrangement, comprising the light source 360 and optionally the reflector 361, 362, 363 ensures consistent light conditions for all image captures.

The enclosure may, but need not, entirely shut out ambient light.

## Claims

1. A method of processing a sample obtained from a livestock animal, comprising:
applying at least some of said sample to a test surface of a growth medium test plate (40),
waiting for a time sufficient to allow microbial growth to form on said test surface,
**characterized by**
acquiring a visual spectrum image depicting at least part of the test surface, using an image capture device, and
providing a computer-implemented pre-trained image classifier algorithm, said image classifier algorithm being pre-trained to determine a microorganism type based on a visible spectrum image depicting a growth pattern of a known microorganism, and
applying said image to the pre-trained image classifier algorithm to determine a microorganism type based on a microorganism growth pattern visible on the image,
wherein the image capture device (111) forms part of a smartphone (11) or a tablet,
wherein the method further comprises positioning the test plate (40) on a first part (32) of an image capture support (30) and positioning the image capture device (111) on a second part (33) of the image capture support (30), said second part being spaced from the first part, and
wherein the acquisition of the image is performed while the image capture device (111) and the test plate (40) are positioned on the image capture support (30).

2. The method as claimed in claim 1, wherein the test plate (40) comprises at least two juxtaposed growth medium regions, said regions differing in at least one of type, color, concentration and composition of the respective growth medium.

3. The method as claimed in claim 2, further comprising:
arranging the test plate (40) with a predetermined orientation relative to the image capture device (111) prior to acquiring said image, such that the growth medium regions present a predetermined orientation in said image; and/or
reorienting the acquired image, such that the growth medium regions present a predetermined orientation in said image.

4. The method as claimed in any one of the preceding claims, wherein said waiting step comprises maintaining the sample in a temperature controlled environment, preferably at a constant temperature of 34-40 degrees C.

5. The method as claimed in any one of the preceding claims, further comprising enclosing the test plate (40) so as to shield it from ambient light and supplying light from a light source, optionally via a reflector.

6. The method as claimed in any one of the preceding claims, further comprising an image limitation step, comprising cropping or masking unwanted portions of the image.

7. The method as claimed in any one of the preceding claims, wherein the pre-trained image classifier algorithm comprises at least one supervised learning algorithm configured and trained to identify at least two microorganism types or microorganism classes.

8. The method as claimed in any one of the preceding claims, wherein the pre-trained image classifier algorithm comprises a plurality of supervised learning algorithms, each of which being configured and trained to identify one microorganism type or microorganism class.

9. The method as claimed in any one of the preceding claims, wherein said applying an image classifier algorithm comprises:
sending the image from the image capture device via a data communication network to a remotely located processing device,
feeding said image to the pre-trained image classifier to obtain a processing result based on the image, and
sending the processing result via the data communication network to the image capture device or to another processing device.

10. The method as claimed in claim 9, wherein the processing result comprises an indication of a microorganism type deemed to be present on the test plate depicted on the image, and optionally a value indicating a confidence level of the processing result.

11. The method as claimed in any one of the preceding claims, further comprising:
waiting for a second time sufficient to allow further microbial growth to form on said test surface,
acquiring a second visual spectrum image of the test surface using an image capture device (111), and
applying the image classifier algorithm to said second image in order to determine a microorganism type based on a microorganism growth pattern visible on the image.

12. The method as claimed in any one of the preceding claims, wherein the sample is a milk sample from a lactating animal.

13. The method as claimed in any one of the preceding claims, wherein the sample is a manure sample from an animal.

14. A system for processing a sample obtained from a livestock animal, comprising:
a growth medium test plate (40),
an image capture support (30)comprising :
a sample holder (34), and
an image capture device holder (35), which is positionable at a predetermined distance from the sample holder (34),
wherein the sample holder (34) is configured to receive a growth medium test plate (40), such that the plate (40) is held at a predetermined position, and
wherein the image capture device holder (35) is configured to receive a smartphone or tablet, positioned and oriented such that a camera of the smartphone is directed towards the sample holder (34);
a user device (11) in the form of a smartphone or tablet comprising an image capture device (111) and a communication device (114), and
a central processing device (10),
wherein the user device (11) is configured acquire a visual spectrum image depicting at least part of a test surface of the growth medium test plate (40), using the image capture device (111), and to send the acquired image to the central processing device (10), and
wherein the central processing device (10) is configured to:
receive the image,
provide a computer-implemented pre-trained image classifier algorithm, said image classifier algorithm being pre-trained to determine a microorganism type based on a visible spectrum image depicting a growth pattern of a known microorganism, and
apply the image to the pre-trained image classifier algorithm to determine a microorganism type based on a microorganism growth pattern visible on the image.

## Patentansprüche

1. Verfahren zum Verarbeiten einer von einem Nutztier erhaltenen Probe, umfassend:
Aufbringen von mindestens einem Teil der Probe auf eine Testfläche einer Testplatte (40) mit einem Wachstumsmedium,
eine ausreichende Zeitlang Warten, bis sich mikrobielles Wachstum auf der Testfläche bilden kann,
**gekennzeichnet durch**
Aufnehmen eines Bilds im sichtbaren Spektrum, das mindestens einen Teil der Testfläche zeigt, unter Verwendung einer Bildaufnahmevorrichtung, und
Vorsehen eines computer-implementierten vortrainierten Bildklassifikationsalgorithmus, wobei der Bildklassifikationsalgorithmus dazu vortrainiert ist, einen Mikroorganismustyp auf Basis eines Bilds im sichtbaren Spektrum zu bestimmen, das ein Wachstumsmuster eines bekannten Mikroorganismus zeigt, und
Anwenden des vortrainierten Bildklassifikationsalgorithmus auf das Bild, um einen Mikroorganismustyp auf Basis eines auf dem Bild sichtbaren Wachstumsmusters des Mikroorganismus zu bestimmen, wobei die Bildaufnahmevorrichtung (111) einen Teil eines Smartphones (11) oder eines Tablets bildet,
wobei das Verfahren ferner das Positionieren der Testplatte (40) auf einem ersten Teil (32) eines Bildaufnahmeträgers (30) und das Positionieren der Bildaufnahmevorrichtung (111) auf einem zweiten Teil (33) des Bildaufnahmeträgers (30) umfasst, wobei der zweite Teil von dem ersten Teil beabstandet ist, und
wobei die Aufnahme des Bilds vorgenommen wird, während die Bildaufnahmevorrichtung (111) und die Testplatte (40) auf dem Bildaufnahmeträger (30) positioniert sind.

2. Verfahren nach Anspruch 1, wobei die Testplatte (40) mindestens zwei nebeneinander liegende Bereiche des Wachstumsmediums umfasst, wobei die Bereiche sich in mindestens einem von dem Typ, der Farbe, der Konzentration und der Zusammensetzung des jeweiligen Wachstumsmediums unterscheiden.

3. Verfahren nach Anspruch 2, ferner umfassend:
Anordnen der Testplatte (40) mit einer vorfestgelegten Orientierung relativ zu der Bildaufnahmevorrichtung (111) vor Aufnehmen des Bilds derart, dass die Bereiche des Wachstumsmediums eine vorfestgelegte Orientierung in dem Bild aufweisen; und/oder
Umorientieren des aufgenommenen Bilds derart, dass die Bereiche des Wachstumsmediums eine vorfestgelegte Orientierung in dem Bild aufweisen.

4. Verfahren nach einem der vorstehenden Ansprüche, wobei der Schritt des Wartens das Halten der Probe in einer temperaturgesteuerten Umgebung umfasst, bevorzugt bei einer konstanten Temperatur von 34 bis 40 Grad C.

5. Verfahren nach einem der vorstehenden Ansprüche, ferner umfassend das Umschließen der Testplatte (40), um sie gegen Umgebungslicht abzuschirmen, und das Zuführen von Licht von einer Lichtquelle, optional über einen Reflektor.

6. Verfahren nach einem der vorstehenden Ansprüche, ferner umfassend einen Bildbegrenzungsschritt, umfassend das Abschneiden oder Abdecken von unerwünschten Teilen des Bilds.

7. Verfahren nach einem der vorstehenden Ansprüche, wobei der vortrainierte Bildklassifikationsalgorithmus mindestens einen überwachten Lernalgorithmus umfasst, der dazu konfiguriert und trainiert ist, mindestens zwei Mikroorganismustypen oder Mikroorganismusklassen zu identifizieren.

8. Verfahren nach einem der vorstehenden Ansprüche, wobei der vortrainierte Bildklassifikationsalgorithmus mehrere überwachte Lernalgorithmen umfasst, von denen jeder dazu konfiguriert und trainiert ist, einen Mikroorganismustyp oder eine Mikroorganismusklasse zu identifizieren.

9. Verfahren nach einem der vorstehenden Ansprüche,
wobei das Anwenden eines Bildklassifikationsalgorithmus umfasst:
Senden des Bilds von der Bildaufnahmevorrichtung über ein Datenkommunikationsnetzwerk zu einer fern angeordneten Verarbeitungseinrichtung,
Zuführen des Bilds zu dem vortrainierten Bildklassifikator, um ein Verarbeitungsergebnis auf Basis des Bilds zu erhalten, und
Senden des Verarbeitungsergebnisse über das Datenkommunikationsnetzwerk zu der Bildaufnahmevorrichtung oder zu einer anderen Verarbeitungseinrichtung.

10. Verfahren nach Anspruch 9, wobei das Verarbeitungsergebnis eine Angabe eines Mikroorganismustyps, von dem angenommen wird, dass er auf der auf dem Bild gezeigten Testplatte vorliegt, und optional eines Werts umfasst, der ein Konfidenzniveau des Verarbeitungsergebnisses angibt.

11. Verfahren nach einem der vorstehenden Ansprüche, ferner umfassend:
eine zweite ausreichende Zeitlang Warten, bis sich weiteres mikrobielles Wachstum auf der Testfläche bilden kann,
Aufnehmen eines zweiten Bilds im sichtbaren Spektrum der Testfläche unter Verwendung einer Bildaufnahmevorrichtung (111), und Anwenden des Bildklassifikationsalgorithmus auf das zweite Bild, um einen Mikroorganismustyp auf Basis eines auf dem Bild sichtbaren Wachstumsmusters des Mikroorganismus zu bestimmen.

12. Verfahren nach einem der vorstehenden Ansprüche, wobei die Probe eine Milchprobe von einem milchgebenden Tier ist.

13. Verfahren nach einem der vorstehenden Ansprüche, wobei die Probe eine Stalldungprobe von einem Tier ist.

14. System zur Verarbeitung einer von einem Nutztier erhaltenen Probe, umfassend:
eine Testplatte (40) mit einem Wachstumsmedium,
einen Bildaufnahmeträger (30) umfassend:
einen Probenhalter (34) und
einen Halter (35) der Bildaufnahmevorrichtung, der in einem vorfestgelegten Abstand von dem Probenhalter (34) positionierbar ist, wobei der Probenhalter (34) dazu konfiguriert ist, eine Testplatte (40) mit einem Wachstumsmedium derart aufzunehmen, dass die Platte (40) in einer vorfestgelegten Position gehalten wird, und
wobei der Halter (35) der Bildaufnahmevorrichtung dazu konfiguriert ist, ein Smartphone oder ein Tablet aufzunehmen, das derart positioniert und orientiert ist, dass eine Kamera des Smartphones auf den Probenhalter (34) gerichtet ist;
eine Benutzervorrichtung (11) in der Form eines Smartphones oder eines Tablets, die eine Bildaufnahmevorrichtung (111) und eine Kommunikationsvorrichtung (114) umfasst, und
eine zentrale Verarbeitungseinrichtung (10),
wobei die Benutzervorrichtung (11) dazu konfiguriert ist, ein Bild im sichtbaren Spektrum aufzunehmen, das mindestens einen Teil einer Testfläche der Testplatte (40) mit einem Wachstumsmedium unter Verwendung der Bildaufnahmevorrichtung (111) zeigt, und das aufgenommene Bild zu der zentralen Verarbeitungseinrichtung (10) zu senden, und
wobei die zentrale Verarbeitungseinrichtung (10) dazu konfiguriert ist:
das Bild zu empfangen,
einen computer-implementierten vortrainierten
Bildklassifikationsalgorithmus vorzusehen, wobei der Bildklassifikationsalgorithmus dazu vortrainiert ist, einen Mikroorganismustyp auf Basis eines Bilds im sichtbaren Spektrum zu bestimmen, das ein Wachstumsmuster eines bekannten Mikroorganismus zeigt, und
den vortrainierten Bildklassifikationsalgorithmus auf das Bild anzuwenden, um einen Mikroorganismustyp auf Basis eines auf dem Bild sichtbaren Wachstumsmusters des Mikroorganismus zu bestimmen.

## Revendications

1. Procédé de traitement d'un échantillon obtenu sur un animal d'élevage, comprenant :
l'application d'au moins une partie dudit échantillon sur une surface de test d'une plaque de test de milieu de croissance (40),
l'attente pendant une durée suffisante pour permettre à la croissance microbienne de se former sur ladite surface de test,
**caractérisé par**
l'acquisition d'une image de spectre visuel représentant au moins une partie de la surface de test en utilisant un dispositif de capture d'images, et
la prévision d'un algorithme de classification d'images pré-entraîné mis en oeuvre par ordinateur, ledit algorithme de classification d'images étant pré-entraîné pour déterminer un type micro-organisme en se basant sur une image de spectre visible représentant un modèle de croissance d'un micro-organisme connu, et
l'application de ladite image audit algorithme de classification d'images pré-entraîné pour déterminer un type de micro-organisme en se basant sur un modèle de croissance de micro-organismes visibles sur l'image,
le dispositif de capture d'images (111) faisant partie intégrante d'un Smartphone (11) ou d'une tablette,
le procédé comprenant en outre le positionnement de la plaque de test (40) sur une première partie (32) d'un support de capture d'images (30) et le positionnement du dispositif de capture d'images (111) sur une seconde partie (33) du support de capture d'images (30), ladite seconde partie étant espacée de la première partie, et
l'acquisition de l'image étant réalisée pendant que le dispositif de capture d'images (111) et la plaque de test (40) sont positionnés sur le support de capture d'images (30).

2. Procédé selon la revendication 1, dans lequel la plaque de test (40) comprend au moins deux zones de milieu de croissance juxtaposées, lesdites zones différant par au moins un paramètre parmi un type, une couleur, une concentration et une composition du milieu de croissance respectif.

3. Procédé selon la revendication 2, comprenant en outre :
la disposition de la plaque de test (40) avec une orientation prédéterminée par rapport au dispositif de capture d'images (111) avant l'acquisition de ladite image, de sorte que les zones de milieu de croissance présentent une orientation prédéterminée dans ladite image ; et/ou
la réorientation de l'image acquise, de sorte que les zones de milieu de croissance présentent une orientation prédéterminée dans ladite image.

4. Procédé selon l'une quelconque des revendications précédentes, dans lequel ladite étape d'attente comprend le maintien de l'échantillon dans un environnement à température contrôlée, de préférence à une température constante de 34 à 40 degrés Celsius.

5. Procédé selon l'une quelconque des revendications précédentes, comprenant en outre le fait d'enclore la plaque de test (40) de manière à l'abriter de la lumière ambiante et de fournir de la lumière d'une source lumineuse, en option via un réflecteur.

6. Procédé selon l'une quelconque des revendications précédentes, comprenant en outre une étape de limite d'images, comprenant le recadrage ou le masquage de sections non voulues de l'image.

7. Procédé selon l'une quelconque des revendications précédentes, dans lequel l'algorithme de classification d'images pré-entraîné comprend au moins un algorithme d'apprentissage supervisé configuré et entraîné pour identifier au moins deux types de micro-organismes ou classes de micro-organismes.

8. Procédé selon l'une quelconque des revendications précédentes, dans lequel l'algorithme de classification d'images pré-entraîné comprend une pluralité d'algorithmes d'apprentissage supervisés, dont chacun est configuré et entraîné pour identifier un type de micro-organisme ou une classe de micro-organismes.

9. Procédé selon la revendication 9, dans lequel ladite application d'un algorithme de classification d'images comprend :
l'envoi de l'image depuis le dispositif de capture d'images via un réseau de communication de données à un dispositif de traitement distant,
l'apport de ladite image dans le classificateur d'images pré-entraîné pour obtenir un résultat de traitement en se basant sur l'image, et
l'envoi du résultat de traitement via le réseau de communication de données au dispositif de capture d'images ou à un autre dispositif de traitement.

10. Procédé selon l'une quelconque des revendications précédentes, dans lequel le résultat de traitement comprend une indication d'un type de micro-organisme présumé présent sur la plaque de test représenté sur l'image, et en option une valeur indiquant un niveau de confiance du résultat de traitement.

11. Procédé selon l'une quelconque des revendications précédentes, comprenant en outre :
l'attente pendant une seconde durée suffisante pour permettre à plus de croissance microbienne de se former sur ladite surface de test,
l'acquisition d'un second spectre visuel de la surface de test en utilisant un dispositif de capture d'images (111), et
l'application de l'algorithme de classification d'images à ladite seconde image afin de déterminer un type de micro-organisme en se basant sur un modèle de croissance de micro-organismes visibles sur l'image.

12. Procédé selon l'une quelconque des revendications précédentes, dans lequel l'échantillon est un échantillon de lait d'un animal allaitant.

13. Procédé selon l'une quelconque des revendications précédentes, dans lequel l'échantillon est un échantillon de fumier d'un animal.

14. Système de traitement d'un échantillon obtenu sur un animal d'élevage, comprenant :
une plaque de test de milieu de croissance (40),
un support de capture d'images (30) comprenant :
un support d'échantillon (34), et
un support de dispositif de capture d'images (35) qui est positionnable à une distance prédéterminée du support d'échantillon (34),
le support d'échantillon (34) étant configuré pour recevoir une plaque de test de milieu de croissance (40), de sorte que la plaque (40) soit maintenue à une position prédéterminée, et
le support de dispositif de capture d'images (35) étant configuré pour recevoir un Smartphone ou une tablette, positionné et orienté de manière à ce qu'une caméra du Smartphone soit dirigée vers le support d'échantillon (34) ;
un dispositif d'utilisateur (11) sous la forme d'un Smartphone d'une tablette comprenant un dispositif de capture d'images (111) et un dispositif de communication (114), et
un dispositif de traitement central (10),
le dispositif d'utilisateur (11) étant configuré pour acquérir une image de spectre visuel représentant au moins une partie d'une surface de test de la plaque de test de milieu de croissance (40) en utilisant le dispositif de capture d'images (111), et pour envoyer l'image acquise au dispositif de traitement central (10), et
le dispositif de traitement central (10) étant configuré pour :
recevoir l'image,
prévoir un algorithme de classification d'images pré-entraîné mis en oeuvre par ordinateur, ledit algorithme de classification d'images étant pré-entraîné pour déterminer un type de micro-organisme en se basant sur une image de spectre visible représentant un modèle de croissance d'un micro-organisme connu, et
appliquer l'image audit algorithme de classification d'images pré-entraîné pour déterminer un type de micro-organisme en se basant sur un modèle de croissance de micro-organismes visibles sur l'image.
